# EUROPEAN PATENT APPLICATION

(11) **EP 3 167 905 A2**
(43) Date of publication of application: **17.05.2017**
(21) Application number: 16002343.8
(22) Date of filing: 03.11.2016
(51) Int. Cl.: A61L 2/18

(54) **BIOLOGICAL SELECTIVE DISINFECTION METHOD UTILIZING BACTERIOPHAGES**

(30) Priority: 05.11.2015 RU 2015147281
(71) Applicant: Limited Liability Company "Pharmassets Capital", Moscow 107045 (RU)
(72) Inventor: Kanygin, Petr Sergeevich, Moscow Region (RU); Akimkin, Vasily Gennadevich, 111250 Moscow (RU); Brusina, Elena Borisovna, 650025 Kemerovo (RU); Drozdova, Olga Michailovna, 650099 Kemerovo (RU); Dabizheva, Alexandra Nikolaevna, 125222 Moscow (RU); Makovsky, Evgeny Victorovich, 119294 Moscow (RU); Achapkina, Svetlana Vladimirovna, 119294 Moscow (RU); Voroshilova, Natalia Nikolaevna, 450077 Ufa (RU); Polygach, Olga Alexandrovna, 450095 Ufa (RU)
(74) Representative: Zellentin & Partner mbB Patentanwälte

(57) **Abstract**

The method is intended for use in the healthcare, in particular for selective disinfection against agents causative of healthcare-associated infections (HCAIs).

To provide the selective disinfection, an agent-specific bacteriophage with a lytic activity of 10⁻⁴ to 10⁻⁹ and phage content of at least 10⁵ per 1 ml of the product is applied in the amount of 1 to 2 ml/m² onto the object to be treated.

The biological selective disinfection method utilizing bacteriophages provides a pronounced effect against Pseudomonasaeruginosa, Salmonellatyphimurium and other most common HCAI agents, does not cause any side effects, is environmentally safe and economically advantageous.

## Description

This invention relates to the healthcare industry, and, more particularly, to disinfection methods (a biological method) utilizing bacteriophages as prophylaxis against healthcare-associated infections (HCAIs).

Healthcare facilities are at high risk of formation of nosocomial clones (strains) of microorganisms that may accumulate at the hospital environment objects and may be responsible for multiple patient morbidity with high mortality rates. The formed nosocomial microorganism clones (strains) often feature antimicrobial resistance, including resistance to antibiotics, chemical sanitizers, and antiseptics.

One currently well-known and widely used method is chemical disinfection. A wide range of chemically active substances (AS), e.g. chlorine-based active substances, oxygen-based active substances, surfactants, aldehydes, alcohols, or various combinations thereof, are used for chemical disinfection. (For example, ref. to Fedorova, L.S., Advanced Disinfectants: Lines of Research in the Areas of Investigation, Assessment and Use. "Epidemiology and Infectious Diseases. Topical Issues" Magazine No. 6, 2013. Pages 19-21; Patents RU Nos. 2308292, 2272652).

Disadvantages of the known chemical disinfection methods are their potential toxic and allergenic effect on the patients and healthcare staff, due to which disinfection must be performed in the absence of humans, or concentrations and quantities of the disinfectant must be limited.

All chemical disinfectants smell. As far as the environment is concerned, chemical disinfectants are environmentally unfriendly and are of broad spectrum, which, under certain conditions, may contribute to nosocomial clone (strain) formation. Microorganisms are capable of developing mechanisms of resistance to any and all known classes of disinfectants.

The object of this invention is to provide a disinfection method that can reduce the incidence of HCAIs and eliminate outbreaks induced by nosocomial strains of HCAI agents at healthcare facilities of various specialties.

The above object is achieved through a biological selective disinfection method, disclosed herein, comprising: application of an agent-specific bacteriophage with a lytic activity of 10⁻⁴ to 10⁻⁹ and phage content of at least 10⁵ per 1 ml of the product in the amount of 1 to 2 ml/m² onto the object to be treated.

Preferably, bacteriophages with a lytic activity of 10⁻⁴ to 10⁻⁹ and phage content of 10⁵ to 10⁹ per 1 ml of the product are used.

Selective disinfection by the biological method utilizing an agent-specific bacteriophage is most useful at healthcare facilities' epidemiologically critical special-purpose departments (such as intensive care and resuscitation units, burns units, surgery departments, etc.), where usage of chemical disinfectants is often restricted due to the impossibility to remove patients from the departments' areas on a regular basis, abundance of complex medical apparatus and patients' vital function monitoring systems in such departments.

In the event of dissemination of a nosocomial clone (strain) of an infectious agent resistant to chemical disinfectants and other antimicrobial treatment, the biological disinfection method utilizing the agent-specific bacteriophage with the above properties is crucial to significantly improving the efficiency of preventive and anti-epidemic measures. Only virulent phages are used in the biological disinfection method.

The biological disinfection method includes using treatment and preventive bacteriophage products that comprise complexes of polyclonal virulent (strictly lytic) bacterial viruses killing homologous bacteria through intracellular multiplication and bacteria cell destruction followed by the release of mature phages capable of infecting new bacterial cells.

Bacteriophages are widely used in the presence of humans, including pre-term infants and pregnant women. Bacteriophages are environmentally resistant and compatible with many chemical disinfectants.

Before using any bacteriophages, testing is required to determine whether an infectious bacterial agent is susceptible to such bacteriophages.

To ensure efficient disinfection by the biological method utilizing an agent-specific bacteriophage, microbiological monitoring shall be performed at the healthcare facility, including dynamic assessment of the microorganisms circulating at the facility and their susceptibility to antibiotics and disinfectants.

Disinfection by the biological method utilizing bacteriophages is indicated, where:
- unfavorable epidemiological situation associated with bacterial infections is detected at a healthcare facility;
- there is high risk of infection onset and spread, the risk being assessed based on the epidemiological analysis and microbiological monitoring results;
- there is evidence of a nosocomial strain (clone) formation;
- any microorganism strains resistant to antibiotics and chemical disinfectants have been detected.

Nosocomial strain (clone) formation evidence include: resistance to antibiotics, resistance to disinfectants, growth of the portion of any bacteria geno- and phenotype in the healthcare facility's microbiological environment and growth of such bacteria type circulation among patients.

The biological selective disinfection method essentially comprises application of an agent-specific bacteriophage with a lytic activity of at least 10⁻⁴ and phage content of at least 10⁵ per 1 ml of the product by aerosol spraying onto potential areas of HCAIs agent accumulation, such as indoor surfaces, objects, and things of high epidemiological risk as infectious agent transmission factors, in the amount of 1 to 2 ml/m².

Specifically, the above areas comprise benches and manipulation tables, medicine cabinet surfaces, infusion stands, breathing equipment, walls, bedstands, ward tables and chairs, wash basins, bed handrails, door handles, etc.

A bacteriophage may be introduced into a humidifier to eliminate an agent from breathing equipment.

Where nosocomial salmonellosis or other acute intestinal bacterial infections have been detected in patients, or where there is high risk of such infections, treatment with the agent-specific bacteriophage must be performed in the patient accommodation wards, toilet, and sanitary rooms.

HCAIs nidus localization and eradication objective is efficiently achieved by introducing minimum quantities of a specific bacteriophage into the environment. Liquid treatment and preventive bacteriophage products, duly registered in the Russian Federation, are used for disinfection.

Treatment and preventive bacteriophages are sterile purified filtrates of the bacteria-specific phage lysates. They are cleared from bacteria metabolism byproducts, endotoxins and exotoxins, bacterial cell phagolysis products, protein and antigen complexes, and culture mediums; they are neither toxic, nor cause allergization. Bacteriophage lytic activity does not depend on bacteria's resistance to antibiotics and chemical disinfectants.

Liquid bacteriophage product in manufacturer's packaging must be transparent and free from any sediment. Bacteriophage bottle opening is done in accordance with aseptic rules. The bottle is shaken before use. Once the bottle is opened, its contents must be used within 2 hours.

The product dose for disinfection by the biological method utilizing an agent-specific bacteriophage is 1 to 2 ml/m².

The best effect is achieved by applying the bacteriophage onto ambient objects by spraying, for which disposable propellant-free aerosol cans may advantageously be used. The product may be diluted with a normal saline solution at the ratio of 1:1 to mitigate the hampering effect of the product foaming.

Disinfection by the biological method utilizing bacteriophages may be performed at any time of day or night, preferably 3 to 4 hours before a routine (final) disinfection with chemical disinfectants or after similar amount of time after completion of such disinfection.

If sprayed appropriately, a fine layer is visible at the bacteriophage application areas. Such manner of phage application provides high rate of bacteriophage circulation in the environment and dramatically increases the likelihood of the bacteriophage contacting with relevant bacteria, thereby ensuring the high efficiency of this bacteriophage-based method.

Quality control of disinfection by the biological method utilizing bacteriophages is done via sanitary and microbiological analysis of washings sampled from the treated surfaces 6 to 8 hours after the disinfection.

Exposure to the specific phage results in the lysis of specific HCAI agents (susceptible to the specific action of the bacteriophage being used), complete elimination of the HCAI agent from the environment (6 to 24 hours), and, consequently, dramatically decreased or no incidence in patients, thus resulting in rapid eradication of long-term nidi of HCAIs.

The biological disinfection method may be advantageously used against gram-negative (Pseudomonas aeruginosa, Klebsiella pneumoniae, Acinetobacter baumannii, Salmonella typhimurium, Shigella flexneri, Proteus mirabilis, Proteus vulgaris, enteropatogennaya Escherichia coli, etc.) and gram-positive (Staphylococcus aureus, Streptococcus spp., etc.) microorganisms causative of HCAIs and resistant to the majority of currently used groups of antibiotics, disinfectants and environmental factors.

### Examples.

### Infection caused by Pseudomonas aeruginosa.

A Pseudomonas aeruginosa specific bacteriophage with a lytic activity of at least 10⁻⁴ and phage content of at least 10⁵ per 1 ml of the product was applied at a resuscitation department in the amount of 1 to 2 ml/m².

In the event of circulation of a multi-resistant nosocomial strain of Pseudomonas aeruginosa resistant to disinfectants, the biological disinfection with a Pseudomonas aeruginosa specific bacteriophage was the only measure providing the sustainable result of complete elimination of the agent from the hospital environment objects.

Where the Pseudomonas aeruginosa specific bacteriophage was used, complete elimination of the agent from the hospital environment objects was achieved within 1 day (24 hours) after a single application with subsequent absence of any new cases of nosocomial Pseudomonas aeruginosa infections.

Use of the Pseudomonas aeruginosa specific bacteriophage in all cases, where the agent was susceptible to it, allowed keeping the in-hospital epidemic situation under full control.

### Infection caused by Klebsiella pneumoniae.

A Klebsiella pneumoniae specific bacteriophage with a lytic activity of at least 10⁻⁴ and phage content of at least 10⁵ per 1 ml of the product was applied at a Department (Neonatal Pathology, Resuscitation and Intensive Care, Burns) in the amount of 1 to 2 ml/m².

Where the Klebsiella pneumoniae specific bacteriophage was used, complete elimination of the agent from the environment within 2 days and a decrease by three times of the incidence in the Neonatal Pathology Department, Resuscitation and Intensive Care Department, and Burns Department patients were achieved.

### Infection caused by Salmonella typhimurium.

A Salmonella-specific bacteriophage with a lytic activity of at least 10⁻⁴ and phage content of at least 10⁵ per 1 ml of the product was applied in the amount of 1 to 2 ml/m².

Where the Salmonella-specific bacteriophage was used, complete elimination of the agent from the environment was achieved within the first two days, and an incidence decrease by 15 times was achieved within the first 5 to 7 days with subsequent zero record of any new cases.

### Infection caused by Staphylococcus aureus.

A Staphylococcus-specific bacteriophage with a lytic activity of at least 10⁻⁵ and phage content of at least 10⁵ per 1 ml of the product was applied in the amount of 1 to 2 ml/m².

Where the Staphylococcus-specific bacteriophage was used, complete elimination of the agent from the environment within 1 day and a decrease by two times of the incidence in the Surgery Departments, Resuscitation and Intensive Care Department, Burns Department, and Neonatal Pathology Department patients were achieved.

Thus, the claimed biological selective disinfection method utilizing bacteriophages is characterized by high efficiency and, in particular, provides a pronounced effect against Pseudomonasaeruginosa, Salmonellatyphimurium and other most common HCAI agents, is not subject to any usage restrictions, does not cause any side effects, is environmentally safe and economically advantageous.

## Claims

1. A biological selective disinfection method, comprising application of an agent-specific bacteriophage with a lytic activity of 10⁻⁴ to 10⁻⁹ and a phage particle content of at least 10⁵ per 1 ml of the product in an amount of 1 to 2 ml/m² onto an object to be treated.

2. Use of a product comprising an agent-specific bacteriophage with a lytic activity of 10⁻⁴ to 10⁻⁹ and a phage particle content of at least 10⁵ per 1 ml of the product for selective disinfection.

3. A product for selective disinfection comprising an agent-specific bacteriophage with a lytic activity of 10⁻⁴ to 10⁻⁹ and a phage particle content of at least 10⁵ per 1 ml of the product.
